# EUROPÄISCHE PATENTSCHRIFT

(11) **EP 1 420 960 B1**
(45) Veröffentlichungstag und Bekanntmachung des Hinweises auf die Patenterteilung: **27.04.2005**
(21) Anmeldenummer: 02774530.6
(22) Anmeldetag: 22.08.2002
(51) Int. Cl.: B41M 5/00

(54) **AUFZEICHNUNGSMATERIAL FÜR DAS TINTENSTRAHL-DRUCKVERFAHREN**
INKJET PRINTING RECORDING MATERIAL
MATERIAU D'IMPRESSION POUR PROCEDE D'IMPRESSION A JET D'ENCRE

(30) Priorität: 24.08.2001 DE 10140677
(43) Veröffentlichungstag der Anmeldung: 26.05.2004
(73) Patentinhaber: Mitsubishi Hitec Paper Flensburg GmbH, 24941 Flensburg (DE)
(72) Erfinder: STORK, Gerhard, 24943 Flensburg (DE); KATO, Makato, Chiba-Ken,277-0863 (JP); LERIUS, Karsten, 24960 Munkbrarup (DE)
(86) Internationale Anmeldenummer: PCT/EP2002/009391
(87) Internationale Veröffentlichungsnummer: WO 2003/018324

(56) Entgegenhaltungen:
- EP-A- 1 016 545
- EP-A- 1 029 703
- WO-A-01/53070

## Beschreibung

Die Erfindung betrifft ein Aufzeichnungsmaterial für das Tintenstrahl-Druckverfahren nach Anspruch 1 mit einem Substrat und einer Tintenempfangsschicht, die auf mindestens eine Seite des Substrates aufgebracht ist, wobei die Tintenempfangsschicht ein anorganisches Pigment und ein Tintenfixiermittel enthält, das mindestens zwei Substanzen umfaßt, von denen die erste Substanz ein Polyethylaminepichlorhydrin und die zweite Substanz ein mehrwertiges Metallsalz ist. Die Erfindung betrifft ferner ein das neue Aufzeichnungsmaterial nutzendes Verfahren zur Aufzeichnung nach dem diskontinuierlichen Tintenstrahldruck-Verfahren.

Auf der Basis von Aminverbindungen und Epihalohydrinen hergestellte Reaktionsprodukte sind als Hilfsmittel für die Tintenempfangsschichten von Tintenstrahl-Aufzeichnungsmaterialien bekannt.
Zur Verbesserung der Wasserbeständigkeit von mittels Tintenstrahl-Druckern erzeugten Druckbildern, die sich durch hervorragende Druckqualität auszeichnen sollen, schlägt beispielsweise die **JP-A-11 277 888** ein Hilfsmittel mit einem linearen kationischen Harz vor, das als Reaktionsprodukt einer ein sekundäres Amin enthaltenden Aminkomponente mit einer anderen mindestens zwei Aminogruppen und ein Epihalohydrin enthaltenden Aminkomponente gebildet ist.
Inhalt der **JP-A-11 277 887** ist ebenfalls ein die Wasserbeständigkeit steigerndes Hilfsmittel mit gegenüber der vorstehend diskutierten Schrift geänderter Strukturformel des linearen kationischen Harzes und vergleichbarer Wirkung, die jedoch It. Beschreibungstext erweitert ist um die Reduzierung des sogenannten "Ink-Bleedings" bei mittels Tintenstrahl-Druckern erzeugten Druckbildern. In beiden vorstehend diskutierten Schriften wird die Viskosität der jeweils das vorgeschlagene Hilfsmittel enthaltenen 20%-tigen wässerigen Lösung mit 50 bis 500 m Pa*s (B-Type; 30°C) angegeben.

Aufgabe der **JP-A-10 152 544** ist es, ein Tintenstrahl-Aufzeichnungspapier zur Verfügung zu stellen, das bei mit wässerigen Tinten erzeugten Druckbildern nur ein geringfügiges "Ink-Bleeding" zuläßt und darüber hinaus eine hohe Farbkonzentration und eine hervorragende Wasserbeständigkeit der Druckbilder ermöglicht. Gelöst werden soll die Aufgabe durch einen Zusatz zur Beschichtungsmasse in Form eines verzweigten kationischen Harzes, das als Reaktionsprodukt von Ammoniak mit mindestens einem bevorzugt primären, sekundären oder tertiären Amin und einem Epihalohydrin vorliegt. Als Amin wird ferner Polyalkylenpolyamin sowie Alkanolamin bevorzugt. Die Viskosität der 10%-tigen wässerigen Lösung dieses kationischen Harzes beträgt 1 bis 30 m Pa*s, gemessen nach Brookfield (60 rpm / 25°C).

Mit dem Ziel, ein Produktionsverfahren für Tintenstrahl-Aufzeichnungspapiere vorzustellen, auf denen die Farbtröpfchen wässeriger Tinte nur gering verlaufen und auf denen Druckbilder mit hoher Farbdichte und hervorragender Wasserresistenz ermöglicht werden, schlägt die **JP-A-09 240 139** den Auftrag eines verzweigten kationischen Harzes vor, der als Reaktionsprodukt eines Polyalkylen-Polyamins mit einem Epihalohydrin und gegebenenfalls ferner mit einer aliphatischen Aminkomponente gebildet ist. Die Viskosität einer das vorgeschlagene kationische Harz beinhaltenden 10%-tigen wässerigen Lösung wird mit 30 m Pa*s angegeben, gemessen nach Brookfield (60 rpm / 25°C).

Den vorne diskutierten Schriften ist als Lehre zu entnehmen, die als Hilfsmittel zu nutzenden kationischen Harze geeigneterweise in mittelmolekular-linearer bzw. niedermolekularverzweigter Form auszuwählen. Dagegen sind diesen Schriften weder Hinweise auf mögliche Mengenverhältnisse der kationischen Harze zu in den Tintenempfangsschichten vorhandenen Pigmenten zu entnehmen, noch daß es vorteilhaft ist, die als Hilfsmittel genutzten kationischen Harze mit Metallsalzen zu verbinden, um verbesserte Eigenschaften für im Tintenstrahldruck-Verfahren zu nutzende Aufzeichnungsmaterialien zu erhalten.

Aus der **JP-A-09 099 630** ist als Tintenfixiermittel in der Tintenempfangsschicht eines Tintenstrahl-Aufzeichnungsmaterials ein Polyamid-Polyamin-Epichlorhydrin bekannt, dessen genauere Struktur genauso wenig wie dessen Molekulargewicht offenbart werden. Als Pigment wird amorphes Siliziumdioxid vorgeschlagen, dessen Teilchengröße nur sehr allgemein mit durchschnittlich in einem Bereich von 6-13 µm liegend angegeben wird.

Entsprechend der **EP-A-0 914 962** soll die hervorragende Tintenstrahl-Aufzeichnungseigenschaft und die überlegene Offset-Bedruckbarkeit eines Aufzeichnungsmaterials mit dessen besonders gut gebundener Oberfläche erreicht werden, die überdies eine hohe Wasserbeständigkeit auszeichnet. Gemäß den Ausführungen dieser Schrift wird die Aufgabe mit einem linearen kationischen Harz in der Tintenempfangsschicht gelöst, wobei als Beispiele dieses kationischen Harzes u.a. Dimethylamin-Epichlorhydrin-Polykondensationsprodukte genannt werden. Neben dem kationischen Harz enthält die Tintenempfangsschicht ferner Bindemittel und gegebenenfalls Pigmente. Der Schrift sind dabei keinerlei Hinweise auf vorteilhafte Eigenschaften der Pigmente in der Tintenempfangsschicht zu entnehmen. Auch offenbart diese Schrift in ihrer Beschreibung keine Lehre zum Mischungsverhältnis von Tintenfixiermittel zu Pigment. Beispielhaft wird ein Verhältnis von 1:10 offenbart, während weitere Beispiele pigmentfreie Tintenempfangsschichten offenbaren.

Schließlich offenbart die **EP-A-0 602 326** ein quaternäres Salz eines linearen Dimethylamin-Epichlorhydrin-Adduktes mit einem zwischen 2 und 2000 liegenden Polymerisationsgrad als Tintenfixiermittel in der Aufzeichnungsschicht eines Tintenstrahlaufzeichnungspapiers. In Kombination mit einem (Meth)-Acrylamiddiallylamin-Copolymer sollen neben einer Erhöhung der Tintenfixierbarkeit Farbabweichungen aufgebrachter Druckbilder vermieden werden.
Sofern überhaupt in den dort offenbarten Aufzeichnungsschichten eingesetzt, kommen als geeignete anorganische und organische Pigmente solche in Betracht, deren Teilchengröße in einem Bereich unterhalb von 4 µm liegt. In der Schrift findet sich kein Hinweis auf die vorteilhafte Kombination des Dimethylamin-Epichlorhydrin-Adduktes mit mehrwertigen Metallsalzen als Tintenfixiermittel.

Auch der Einsatz von zwei- oder höherwertigen Metallsalzen in der Aufzeichnungsschicht von Tintenstrahl-Aufzeichnungsmaterialien ist bekannt und wird beispielsweise ohne Kombination mit anderen tintenfixierverbessernden Mitteln in der **DE-A-25 33 957** und der **DE-A-24 01 866** vorgeschlagen. Grundsätzlich hat jedoch der alleinige oder vorherrschende Einsatz von Metallsalzen als Tintenfixiermittel in Tintenstrahl-Aufzeichnungsschichten den Nachteil, daß eine farbgetreue Wiedergabe aufgebrachter Tintendruckbilder kaum gelingt.

Aus der **EP-A-1 016 545** ist ein Tintenstrahldruck-Aufzeichnungsmaterial bekannt, bei dem mittels einer Bindemittelmischung für die Bildempfangsschicht, die in einem bestimmten Verhältnis Polyethylenglycol und Polyvinylalkohol enthält, Mattglanz, höhere Tintenabsorption, Bildqualität, Wasserfestigkeit, Lichtbeständigkeit und Farbübertragung sowie Widerstand gegenüber Verlaufen der Tinte (englisch: ink-bleeding) erzielt werden sollen.
Die Bildempfangsschicht kann mehrwertige Metallsalze und ein aus der Gruppe der polymeren quaternären Ammoniumverbindungen oder Basispolymere wie beispielsweise Poly(dimethylaminoethyl)-methacrylate, Polyalkylenpolyamine sowie deren Kondensationsprodukte mit Dicyanodiamide und Aminepichlorhydrin-Polykondensate enthalten. Die Struktur und das Molekulargewicht dieser Verbindungen sind nicht offenbart. Die Bildempfangsschicht enthält zur Verdichtung offener poröser Strukturen kolloidale Oxide, wie z.B. kolloidales Siliziumdioxid oder mit Aluminiumoxid modifiziertes Siliziumdioxid ohne daß vorteilhafte Mengenverhältnisse offenbart werden.

Die **DE 34 33 528 C2** schlägt schließlich ein Aufzeichnungsmaterial für den Tintenstrahldruck mit einem Träger vor, der zumindest im Oberflächenbereich ein wasserlösliches Metallsalz eines Metalls mit einer lonenwertigkeit von 2 bis 4 und ein kationisches organisches Material, ausgewählt unter Alkylaminsalzen, quaternären Ammoniumsalzen, Polyaminen und basischen Latices aus der Gruppe der Polyaminlatices und Alkylammoniumlatices enthält. Als Beispiel der Polyamine wird u.a. Polyethylaminepichlorhydrin genannt, wobei der Schrift jedoch weder weitere Hinweise über dessen besonders geeignete Vertreter noch über besondere Ausgestaltungen spezieller Vertreter von Polyethylaminepichlorhydrin zu entnehmen sind. Genauso wenig ist der Schrift eine Lehre über vorteilhafte Eigenschaften der einzusetzenden Pigmente wie zum Beispiel hinsichtlich der Teilchengröße zu entnehmen.

Lange Zeit wurden Aufzeichnungstinten auf Pigmentbasis anstelle von Tinten auf Basis organischer Farbstoffe, insbesondere von sauren Azofarbstoffen, kaum verwendet. Problematisch ist bei dieser Art von Aufzeichnungstinten die geringe Lichtbeständigkeit der organischen Farbstoffe, verbunden mit dem Problem verblassender und sich verfärbender Druckbilder, das bislang nur unbefriedigend mittels UV-Absorbern in den Aufzeichnungstinten und mittels Farbstabilisatoren in den Tintenstrahl-Aufzeichnungs-materialien gelöst werden konnte.
Die inzwischen häufiger eingesetzten Aufzeichnungstinten auf Pigmentbasis sind wesentlich lichtstabiler als die zuvor diskutierten Tinten auf Basis organischer Farbstoffe, jedoch besteht bei den Aufzeichnungstinten auf Pigmentbasis das Problem des "Ink-Bleedings", worunter das Verlaufen von aneinander direkt angrenzenden, unterschiedlich farbiger Druckmuster ineinander direkt nach dem Druckvorgang zu verstehen ist. Das erfindungsgemäße Aufzeichnungsmaterial ist für den Einsatz mit Aufzeichnungstinten auf Pigmentbasis entwickelt und reduziert das Problem des "Ink-Bleedings" signifikant.

Beim Tintenstrahldruck wird grundsätzlich zwischen zwei unterschiedlichen Verfahren der Tröpfchenerzeugung unterschieden.

Das kontinuierliche Verfahren sieht einen unter Druck aus einer Düse ausgestoßenen Tintenstrahl vor, der sich aufgrund der Oberflächenspannung in einem gewissen Abstand von der Düse in sehr kleine Tröpfchen aufspaltet. Die Tröpfchen werden elektrisch aufgeladen und anschließend in Abhängigkeit von dem zu erzeugenden Druckbild durch das elektrische Feld elektronisch angesteuerter Ablenkplatten entweder in einen Auffangbehälter abgelenkt oder auf das Aufzeichnungsmaterial plaziert.

Beim diskontinuierlichen, sogenannten "Drop-on-demand"-Verfahren werden die Tintentröpfchen in Abhängigkeit von dem zu erzeugenden Druckbild nur dann erzeugt und aus einer Düse ausgestoßen, wenn auf dem Aufzeichnungsmaterial ein Bildpunkt zu erzeugen ist. Die eine Art von "Drop-on-demand"-Druckern nutzt dabei den piezoelektrischen Effekt, in dem ein elektrisch angesteuertes Piezoelement ein Tintentröpfchen vom Reservoir der Aufzeichnungstinte separiert und dieses Tröpfchen aus einer Düse herausstößt. Im Gegensatz dazu nutzen "BubbleJet"-Drucker ein elektrisch angesteuertes Heizelement, das kleinste Mengen einer wässerigen Tinte in einer Dampfblase entstehen läßt. Der resultierende Dampfdruck stößt das Tröpfchen aus.
Die Erfindung bezieht sich auf Aufzeichnungsmaterialien, die in beiden diskontinuierlichen Verfahren verwendet werden können.

Es ist Aufgabe der vorliegenden Erfindung, ein unter wirtschaftlichen Gesichtspunkten kostengünstiges Aufzeichnungsmaterial für das Tintenstrahl-Druckverfahren zur Verfügung zu stellen, das besonders geeignet ist, mit Aufzeichnungstinten auf Pigmentbasis im diskontinuierlichen Verfahren bedruckt zu werden. Dabei soll das neue Aufzeichnungsmaterial eine hervorragende Fixierung der aufgebrachten Tinten garantieren und so das sogenannte "Ink-Bleeding" reduzieren. Mit der Ausrichtung des neuen Aufzeichnungsmaterials auf pigmentierte Tinten soll eine hohe Lichtbeständigkeit der aufgebrachten Druckbilder ermöglicht werden.
Da pigmentierte Tinten vorzugsweise im Posterdruck eingesetzt werden und die so geschaffenen Poster beispielsweise als Werbetafeln direkter Sonneneinstrahlung ausgesetzt sind, ist bevorzugt ein neues Aufzeichnungsmaterial zur Verfügung zu stellen, daß sich durch eine große Stabilität gegenüber jeglicher Vergilbungsneigung auszeichnet. Im gleichen Maße soll das Aufzeichnungsmaterial mindestens eine akzeptable Nasswischfestigkeit aufgebrachter Druckbilder sicherstellen.

In Abkehr von den bisher auf diesem technischen Gebiet offenbarten Lehren erkannten die Erfinder als Ergebnis zahlreicher Produktions- und Vergleichsversuche, daß die Aufgabe durch ein Aufzeichnungsmaterial für das Tintenstrahl-Druckverfahren gelöst wird mit einem Substrat und einer Tintenempfangsschicht, die auf mindestens eine Seite des Substrates aufgebracht ist, wobei die Tintenempfangsschicht ein anorganisches Pigment und ein Tintenfixiermittel enthält, das mindestens zwei Substanzen umfaßt, von denen die erste Substanz ein Polyethylaminepichlorhydrin und die zweite Substanz ein mehrwertiges Metallsalz ist, wobei
- die erste Substanz ein mittelmolekular verzweigtes Polyamin-Epichlorhydrin-Kondensationsprodukt ist,
- das Verhältnis der ersten Substanz zur zweiten Substanz zwischen 3 : 1 und 16 : 1 liegt,
- das Pigment zu mindestens 80 Gew.-% mit einer D₅₀-Teilchengröße (Malvern) in einem Bereich von 4 µm einschließlich bis 12 µm einschließlich vorliegt und
- das Verhältnis Tintenfixiermittel zu Pigment in einem Bereich von einschließlich 1 : 2 bis einschließlich 1 : 6 liegt.

Im Sinne dieser Erfindung gilt das Polyamin-Epichlorhydrin-Kondensationsprodukt als mittelmolekular, sofern es als 10%-tige wässerige Lösung eine Viskosität in einem Bereich von 15 m Pa*s bis 50 m Pa*s aufweist. Die vorgenannte Viskosität wird dabei nach Brookfield (Spindel 1 bei 100 rpm und bei 25°C) bestimmt.

Grundlage der Erfindung ist die Wahl des Polyamin-Epichlorhydrin-Kondensationsproduktes als erste Substanz des mindestens zwei Substanzen umfassenden Tintenfixiermittels. Es ist erfindungswesentlich, daß das Polyamin-Epichlorhydrin-Kondensationsprodukt in mittelmolekularer verzweigter Form vorliegt. Andere Ausführungsarten des Polyamin-Epichlorhydrin-Kondensationsproduktes erweisen sich aus unterschiedlichen Gründen als ungeeignet; so ist beispielsweise von der Verwendung eines niedermolekularen nicht-verzweigten Polyamin-Epichlorhydrin-Kondensationsproduktes abzusehen, weil solches enthaltende Aufzeichnungsmaterialien eine zu starke Geruchsbildung durch das freie Amin aufweisen.
Als zweite Substanz muß das Tintenfixiermittel ein mehrwertiges Metallsalz umfassen, damit eine Lösung der dieser Erfindung zugrundeliegenden Aufgabe ermöglicht wird.

Durch im Rahmen umfangreicher Versuchsreihen vorgenommene Vergleiche erkannten die Erfinder, dass nur bei einem Verhältnis (atro) der ersten Substanz zur zweiten Substanz zwischen 3 : 1 und 16 : 1 eine gute Tintenfixierung mit minimiertem "Ink-Bleeding"-Verhalten bei gleichzeitig akzeptabler Nasswischfestigkeit auftritt. Als bevorzugter Bereich gilt ein Verhältnis (atro) der ersten Substanz zur zweiten Substanz zwischen 6 : 1 und 14: 1 mit hervorragender Tintenfixierung und stark minimiertem "Ink-Bleeding"-Verhalten bei gleichzeitig besonders überzeugender Nasswischfestigkeit.
Als zweite Substanz des Tintenfixiermittels bieten sich dabei insbesondere Magnesium-Chlorid, Aluminium-Chlorid und ganz besonders bevorzugt Calciumchlorid als Vertreter der mehrwertigen Metallsalze an.

Das erfindungsgemäße Verhältnis Tintenfixiermittel zu Pigment liegt in einem Bereich von 1 : 2 bis 1 : 6 ― bezogen auf die Gewichtsprozente von Pigment und Tintenfixiermittel in der Tintenempfangsschicht. Es wird einerseits beschränkt durch eine verstärkt abnehmende Nasswischfestigkeit, die bei größeren Mengen an Tintenfixiermittel jenseits eines Tintenfixiermittel-Pigment-Verhältnisses von 1 : 2 nicht mehr zu akzeptieren ist, und andererseits durch ein zunehmend schlechter werdendes "Ink-Bleeding"-Verhalten, das bei kleineren Mengen an Tintenfixiermittel jenseits eines Tintenfixiermittel-Pigment-Verhältnisses von 1 : 6 als nicht mehr überzeugend abzulehnen ist.
Entsprechend den Ergebnissen der dieser Erfindung zugrunde liegenden Versuchsreihen erkannten die Erfinder, dass besonders gute Eigenschaften des neuen Aufzeichnungsmaterials vorliegen, wenn das Tintenfixiermittel-Pigment-Verhältnis in einem Bereich von einschließlich 1 : 3 bis einschließlich 1 : 5,5 ― bezogen auf die Gewichtsprozente von Pigment und Tintenfixiermittel in der Tintenempfangsschicht ― liegt, wobei sich erneut die Gewichtsprozente auf alle in der Tintenempfangsschicht eingebundenen Tintenfixiermittel bzw. Pigmente beziehen.

Für die Erfinder völlig überraschend war es, daß die dieser Erfindung zugrundeliegende Aufgabe jedoch nur dann vollständig und überzeugend gelöst werden kann, wenn gleichzeitig zu den vorstehend ausgeführten Merkmalen das anorganische Pigment zu mindestens 80 Gew.-% mit einer Teilchengröße in einem Bereich von 4 µm einschließlich bis 12 µm einschließlich und besonders vorteilhaft von 5 µm bis 12 µm einschließlich vorliegt, bestimmt als D₅₀-Wert (Malvern ― das heißt, die Bestimmung erfolgt lt. Angaben der Pigmenthersteller unter Verwendung der Methode nach Malvern). Als ganz besonders bevorzugt gilt dabei ein Bereich zwischen 6 und 12 µm, bestimmt als D₅₀-Wert (Malvern).

In Vergleichsuntersuchungen zeigte sich, daß bei dem alleinigen oder ausschließlich mit einem mehrwertigen Metallsalz kombinierten Einsatz von Polydiallyl-Dimethyl-Ammonium-Chlorid (Poly-DADMAC) als Tintenfixiermittel das "Ink-Bleeding"-Verhalten bedeutend schlechter ist. Der Einsatz von Polyethylenimin bzw. Polydicyandiamiden verbietet sich aufgrund einer vergrößerten Vergilbungsneigung von Aufzeichnungsmaterialien, die diese kationischen Polymere aufweisen.

Erfindungsgemäß besteht das Tintenfixiermittel des erfindungsgemäßen Aufzeichnungsmaterials lediglich aus dem als erste Substanz einzusetzenden Polyamin-Epichlorhydrin-Kondensationsprodukt und der aus einem mehrwertigen Metallsalz bestehenden zweiten Substanz. Daneben kann das Tintenfixiermittel jedoch eine oder mehrere weitere zur Tintenfixierung übliche Verbindungen, wie beispielsweise Polydiallyl-Dimethyl-Ammonium-Chlorid, kationische Polyacrylamide, kationische Polyacrylate, Polyvinylamine, Polyethylenimin und Polydicyandiamiden oder auch ein niedermoleklar nicht-verzweigtes Polyamin-Epichlorhydrin-Kondensationsprodukt enthalten, sofern sie bezogen auf die Gesamtmenge des Tintenfixiermittels in einer 30 Gew.-%, insbesondere in einer 10 Gew.-%, nicht überschreitenden Menge vorliegen.

Es hat sich als vorteilhaft erwiesen, daß der Gesamt-Anteil von Tintenfixiermittel in der Tintenempfangsschicht bevorzugt in einem Bereich zwischen 5 und 20 Gew.-% liegt, wobei dieser Gesamtanteil beim Auftrag von Tintenempfangsschichten mit hoher flächenbezogener Masse innerhalb des obigen Bereichs tendenziell eher zur unteren Grenze gewählt werden kann.

Der Pigmentanteil der Tintenempfangsschicht des erfindungsgemäßen Aufzeichnungsmaterials liegt vorzugsweise in einem Bereich zwischen 30 und 70 Gew.-%. Dabei haben sich besonders Aluminiumhydroxid sowie Kiesel(säure)gel und Fällungskieselsäure gut bewährt. Die drei zuletzt aufgeführten Pigmente können dabei jeweils mit Aluminium oder mit Aluminiumoxid modifiziert wie auch unmodifiziert ausgebildet sein.
Die zuvor aufgeführten anorganischen Pigmente können jeweils für sich allein oder in Kombination untereinander oder/und in Kombination mit weiteren anorganischen Pigmenten den Pigmentanteil der Tintenempfangsschicht des erfindungsgemäßen Aufzeichnungsmaterials darstellen.

Die Tintenempfangsschicht des erfindungsgemäßen Aufzeichnungsmaterials umfaßt vorzugsweise einen Anteil aus Bindemittel und Co-Bindemittel in einem Bereich von 10 bis 55 Gew.-%. Besonders bewährt haben sich dabei wässerige Polymer-Dispersionen von Ethylen-Vinylacetat sowie insbesondere Styrolbutadienlatex, Polyacrylate sowie Lösungen von teil- oder vollständig verseiftem Polyvinylalkohol, die allein oder auch in Kombination eingesetzt werden.

Neben den vorne stehend diskutierten Inhaltsstoffen kann die Tintenempfangsschicht des erfindungsgemäßen Aufzeichnungsmaterials weitere Komponenten wie beispielsweise Natronlauge, optische Aufheller und Entschäumer, ohne darauf beschränkt zu sein, enthalten. Sie sind nach Notwendigkeit zugegeben und machen bis zu 5 Gew.-% an der Tintenempfangsschicht aus, wobei sich die einzelnen Anteile zu 100 Gew.-% ergänzen.

Um dem Anforderungsspektrum überzeugend gerecht zu werden, sollte die flächenbezogene Masse der Tintenempfangsschicht nicht zu gering gewählt werden, da sonst die Nasswischfestigkeit zu stark abnimmt und genauso das "Ink-Bleeding" in einem zu geringen Maße reduziert werden kann. Nach oben ist die flächenbezogene Masse der Tintenempfangsschicht in erster Linie aus wirtschaftlichen Gesichtspunkten begrenzt.

In zahlreichen Versuchen zeigte es sich als vorteilhaft, die Tintenempfangsschicht in zwei übereinander positionierten Lagen auszubilden, wobei eine untere Lage mit dem Substrat oder mit einer auf das Substrat aufgebrachten Präparationsschicht gemäß einer bevorzugten Ausführungsform in Verbindung steht während eine obere Lage auf die untere Lage aufgebracht ist. Besonders gute Ergebnisse können erzielt werden, sofern die erste Lage eine flächenbezogene Masse zwischen 4 und 12 g/m², bevorzugt zwischen 6 und 8 g/m², und die zweite Lage eine flächenbezogene Masse zwischen 2 und 10 g/m², bevorzugt zwischen 4 und 6 g/m² aufweist. Für die beiden Lagen gelten grundsätzlich die gleichen Lehren hinsichtlich der erfindungswesentlichen Komponentenauswahl und dem Verhältnis dieser Komponenten zueinander, insbesondere auch in ihren bevorzugten Ausführungsformen. Die beiden Lagen weisen auch die gleichen Hilfskomponenten entsprechend den dieser Schrift zu entnehmenden Offenbarungen auf.
Wird die Tintenempfangsschicht nur einlagig ausgebildet, empfiehlt sich eine flächenbezogene Masse dieser Schicht zwischen 4 und 18 g/m², wobei besonders ein Bereich zwischen 5 und 10 g/m² und ganz besonders ein Bereich zwischen 7 und 9 g/m² bevorzugt wird.

Die Feststoffgehalte und Viskositäten der Streichmassen zur Ausbildung einer einzigen oder in bevorzugter Ausführungsform zweier Lagen der erfindungswesentlichen Tintenempfangsschicht sind den anzuwendenden Streichverfahren angepaßt.
Grundsätzlich ist die Erfindung hinsichtlich zu verwendender Streichwerke zum Auftragen der Tintenempfangsschicht nicht beschränkt; besonders bieten sich egalisierende Streichverfahren wie Rollrakel oder Blade sowie Konturstreichverfahren wie Düsen- oder bevorzugt Vorhangbeschichter und Luftbürste an ohne darauf beschränkt zu sein.
Wird die Tintenempfangsschicht in zwei Lagen aufgetragen, ist es besonders vorteilhaft, die erste Lage mit einem egalisierenden Streichverfahren wie Rollrakel oder Blade, in bevorzugter Ausführungsform on-line innerhalb der Papiermaschine, und die zweite Lage mit einem Konturstreichverfahren, bevorzugt mit einem Vorhangbeschichter oder einer Luftbürste, die innerhalb einer separaten Streichmaschine integriert sein können, aufzutragen.

Es wird bevorzugt, daß zwischen Substrat und der einzigen bzw. in bevorzugter Ausführungsform der ersten Lage der Tintenempfangsschicht mindestens eine Präparationsschicht angeordnet ist, die eine flächenbezogene Masse bevorzugt in einem Bereich von 0,5 bis 2 g/m² aufweist. Die Präparationsschicht kann als einfacher Stärkeauftrag ausgebildet sein.

In einer besonders bevorzugten Ausführungsform umfaßt das erfindungsgemäße Aufzeichnungsmaterial auf der Seite, die der Seite mit der Tintenempfangsschicht gegenüberliegt, eine Rückseitenbeschichtung, die als Tintenempfangsschicht, aber auch als einfacher Stärkeauftrag ausgebildet sein kann. Gerade als Stärkeauftrag dient er dazu, eine gute Griffigkeit zu gewährleisten, damit keine Transportschwierigkeiten in den Tintenstrahldruckern zu beklagen sind. Auch ist mit Hilfe eines einfachen Stärkeauftrags eine verbesserte Bedruckbarkeit insbesondere im Offset-Druckverfahren zu erzielen. Der als Rückseitenbeschichtung ausgebildete Stärkeauftrag weist bevorzugt eine flächenbezogene Masse in einem Bereich von 0,1 bis 2,0 g/m² auf.

Die Erfindung umfaßt ferner ein Verfahren zur Aufzeichnung nach dem diskontinuierlichen Tintenstrahldruck-Verfahren mit einer Aufzeichnungstinte auf Pigmentbasis und mit einem erfindungsgemäßen Aufzeichnungsmaterial, das in einer der vorne stehend beschriebenen bevorzugten Ausführungsform ausgebildet sein kann. Das neue Verfahren sieht das Bedrucken des erfindungsgemäßen Aufzeichnungsmaterials mit "Drop-on-demand"-Druckern vor, die entweder den piezoelektrischen Effekt oder als "BubbleJet"-Drucker ein elektrisch angesteuertes Heizelement zur Ausstoßung kleinster Tintentröpfchen nutzen.

Die in Beschreibung und Patentansprüchen gemachten Angaben zur flächenbezogenen Masse, zu Gew.-% (Gewichts-%), zu Gew.-Teilen (Gewichts-Teilen) und zu Komponentenverhältnissen beziehen sich, soweit nicht ausdrücklich anders vermerkt, jeweils auf das "atro"-Gewicht, d.h. absolut trockene Gewichtsteile. Die Abkürzung "lutro" steht für lufttrocken, und bedeutet, sofern genutzt, daß die so gekennzeichneten Komponenten in ihrer handelsüblichen Lieferform beschrieben werden.

Folgende Beispiele und Vergleichsbeispiele werden die Erfindung weitergehend verdeutlichen:

### Herstellung eines Substrats:

Auf einer Langsieb-Papiermaschine wird als Substrat eine Papierbahn aus gebleichten und gemahlenen Laub- und Nadelholzzellstoffen unter Zugabe üblicher Beischlagstoffe in üblichen Mengen mit einer flächenbezogenen Masse von 82 g/m² hergestellt. Vorderseitig erhält die Papierbahn eine Stärkepräparation von 0,8 g/m², rückseitig wird eine Stärkepräparation von 0,4 g/m² aufgetragen.

### Grundrezeptur 1 zur Herstellung von Streichmassen für eine Tintenempfangsschicht zu den erfindungsgemäßen Beispielen 1, 5, 6 und 7 sowie zu den Vergleichsbeispielen 2, 3, 4, 8 und 9:

In einem Behälter werden, bezogen auf insgesamt 500 Gew.-Teile (lutro), unter ständigem Rühren folgende Komponenten eingegeben:

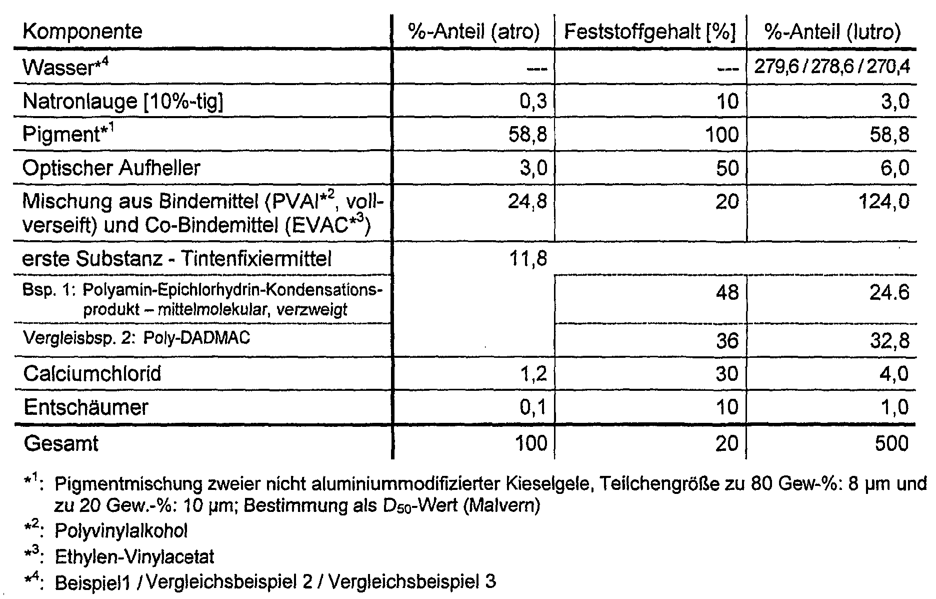

Als Tintenfixiermittel wird in der Streichmasse zu Beispiel 1 eine Komposition aus einem mittelmolekularen verzweigten Polyamin-Epichlorhydrin-Kondensationsprodukt und Calciumchlorid in einem Verhältnis Polyamin-Epichlorhydrin-Kondensationsprodukt zu Calciumchlorid von 9,8 : 1 eingesetzt.

Dagegen weist die Streichmasse gemäß Vergleichsbeispiel 2 bei ansonsten gleichen Komponenten im Vergleich zu Beispiel 1 statt des Polyamin-Epichlorhydrin-Kondensationsproduktes ein Polydiallyl-Dimethyl-Ammonium-Chlorid auf. Zur Ausbildung der einlagigen Tintenempfangsschichten werden die Streichmassen auf die Vorderseite der in ihrer Herstellung vorstehend beschriebenen Papierbahn mit 8,5 g/m² aufgetragen. Auf diese Weise entsteht jeweils eine Probe des erfindungsgemäßen Beispiels 1 sowie des Vergleichsbeispiels 2.

Zur Untersuchung des "Ink-Bleeding"-Verhaltens wird unter Verwendung eines Tintenstrahldruckers, Typ HP-DesignJet 2500 CP, der Firma Hewlett Packard und unter Benutzung von UV-Tinten des gleichen Herstellers jeweils ein Kontrolldruck auf die beiden Proben aufgebracht (Druckmodus: normal ohne Farbanpassung, Druckertreiber: gestrichene Papier, schwer). Entsprechend einer visuellen Beurteilung ist das "Ink-Bleeding"-Verhalten der Proben von Beispiel 1 gut, dagegen ist das "Ink-Bleeding"-Verhalten der Proben von Vergleichsbeispiel 2 als schlecht zu bewerten.

Für die nachfolgenden Beispiele 5, 6 und 7 und Vergleichsbeispiele 3, 4, 8 und 9 werden Streichmassen gemäß der Atro-Anteile von Beispiel 1 hergestellt und entsprechend Beispiel 1 auf eine in ihrer Herstellung vorstehend beschriebene Papierbahn mit einer flächenbezogenen Masse gemäß Tabelle 1 aufgetragen. Im Vergleich zu Beispiel 1 wird das Verhältnis des mittelmolekularen verzweigten Polyamin-Epichlorhydrin-Kondensationsprodukt zu Calciumchlorid variiert.

Erneut wird zur Untersuchung des "Ink-Bleeding"-Verhaltens ein Tintenstrahldrucker, Typ HP-DesignJet 2500 CP, der Firma Hewlett Packard (Druckmodus: normal ohne Farbanpassung, Druckertreiber: gestrichene Papier, schwer) verwendet unter Benutzung von UV-Tinten des gleichen Herstellers. Dazu wird jeweils ein Kontrolldruck auf die Proben aufgebracht und das "Ink-Bleeding"-Verhalten der Proben visuell beurteilt.

Zur Untersuchung dieser Nasswischfestigkeit werden die Druckbilder, an denen zuvor das "Ink-Bleeding"-Verhalten der Proben beurteilt wurde, mit Wasser beträufelt. Nach 2 Sekunden Einwirkzeit wischt man mehrmals mit einem Finger unter gleichbleibendem Druck über das Druckbild. Dabei sollte das Druckbild möglichst wenig, im Idealfall überhaupt nicht verwischt werden.
In Tabelle 1 sind erfindungsgemäße Beispiele mit "EB" und Vergleichsbeispiele mit "VB" bezeichnet. Die ermittelten Meßwerte und Beurteilungen ergeben sich aus Tabelle 1:

**Tabelle 1:**

| Beispiel | Verhältnis Amin-Epichlorhydrin-Kondensationsprodukt zu Calciumchlorid | Viskosität der Streichmasse [Brookfield (Spindel 2 / 50 rpm / 25°C)] | Flächenbezogene Masse der Tintenempfangsschicht [g/m²] | "Ink-Bleeding"-Verhalten | Nasswischfestigkeit |
|---|---|---|---|---|---|
| 3 VB | 1 : 5 | 744 | 8,4 | Gut | Schlecht |
| 4 VB | 1,5 : 1 | > 2000 | 7,9 | Gut | Schlecht |
| 5 EB | 3 : 1 | 360 | 8,4 | Gut | Befriedigend |
| 6 EB | 9 : 1 | 336 | 8,2 | Gut | Gut |
| 7 EB | 12,5 : 1 | 352 | 8,2 | Gut | Gut |
| 8 VB | 20 : 1 | 368 | 8,3 | Unbefriedigend | Gut |
| 9 VB | 100 : 0 (kein Calciumchlorid | 368 | 8,4 | Schlecht | Gut |

### Grundrezeptur 2 zur Herstellung von Streichmassen für eine Tintenempfangsschicht zu den erfindungsgemäßen Beispielen 11, 14, 17 und 20 und zu den Vergleichsbeispielen 10, 12, 13, 15, 16, 18, 19 und 21 bis 24:

In einem Behälter werden, bezogen auf insgesamt 500 Gew.-Teile (lutro), unter ständigem Rühren folgende Komponenten eingegeben:

| Komponente | %-Anteil (atro) | Feststoffgehalt [%] | %-Anteil (lutro) |
|---|---|---|---|
| Wasser | --- | --- | 279,6 |
| Natronlauge [10%-tig] | 0,3 | 10 | 3,0 |
| Pigment | 58,8 | 100 | 58,8 |
| Optischer Aufheller | 3,0 | 50 | 6,0 |
| Mischung aus Bindemittel (PVAI*¹, vollverseift) und Co-Bindemittel (EVAC*²) | 24,8 | 20 | 124,0 |
| Polyamin-Epichlorhydrin-Kondensationspr. | 11,8 | 50 | 23,6 |
| Calciumchlorid | 1,2 | 30 | 4,0 |
| Entschäumer | 0,1 | 10 | 1,0 |
| Gesamt | 100 | 20 | 500 |

| | | | |
|---|---|---|---|
| *¹: Polyvinylalkohol | | | |
| *²: Polymer aus Ethylen-Vinylacetat | | | |

Als Pigment wurde nicht aluminiummodifiziertes Kieselgel mit einem Porenvolumen von 1,2 ml/g bei folgenden mittleren Teilchengrößen und spezifischen inneren Oberflächen eingesetzt:

| | Pigment 1 | Pigment 2 | Pigment 3 | Pigment 4 | Pigment 5 |
|---|---|---|---|---|---|
| Teilchengröße, [µm] Bestimmung als D₅₀-Wert (Malvern) | 4 | 6,5 | 8 | 10 | 15 |
| Spezifische innere Oberfläche [m²/g] | 290 | 390 | 290 | 390 | 175 |

Die Beispiele der Grundrezeptur 2 sehen als Tintenfixiermittel eine Komposition vor, die als erste Substanz jeweils ein Polyamin-Epichlorhydrin-Kondensationsprodukt mit folgender Unterscheidung umfaßt:
- Tintenfixiermittel A: mittelmolekular, nicht verzweigt (36,5 m Pa*s)
- Tintenfixiermittel B: mittelmolekular, verzweigt (35 m Pa*s)
- Tintenfixiermittel C: hochmolekular, nicht verzweigt (91 m Pa*s)
Das Tintenfixiermittel besteht außerdem aus Calciumchlorid als zweite Substanz, das in einem Verhältnis Polyamin-Epichlorhydrin-Kondensationsprodukt zu Calciumchlorid von 9,8 : 1 eingesetzt wird.

Die in vorstehender Aufzählung der Tintenfixiermittel in Klammern stehenden Zahlen geben die Viskosität des Polyamin-Epichlorhydrin-Kondensationsproduktes als 10%-tige wässerige Lösung wieder, gemessen nach Brookfield (Spindel 1 bei 100 rpm und bei 25°C).

### Beispiele 11, 14, 17, 20 und Vergleichsbeispiele 10, 12, 13, 15, 16, 18, 19 und 21 bis 24:

Es wurden 15 Proben unterschiedlicher Aufzeichnungsmaterialien für das Tintenstrahl-Druckverfahren hergestellt.
Dazu wurden 15 unterschiedliche Streichmassen entsprechend der obigen Grundrezeptur angesetzt, wobei jedes der fünf eingeführten Pigmente 1, 2, 3, 4 und 5 mit jedem der Tintenfixiermittel, umfassend A, B oder C zusammen mit Calciumchlorid, kombiniert wird. Zur Ausbildung der Tintenempfangsschichten werden die Streichmassen auf die in ihrer Herstellung vorstehend beschriebenen Papierbahn aufgetragen.
Erneut wird zur Untersuchung des "Ink-Bleeding"-Verhaltens ein Tintenstrahldrucker, Typ HP-DesignJet 2500 CP, der Firma Hewlett Packard (Druckmodus: normal ohne Farbanpassung, Druckertreiber: gestrichene Papier, schwer) verwendet unter Benutzung von UV-Tinten des gleichen Herstellers. Dazu wird jeweils ein Kontrolldruck auf die 20 Proben aufgebracht und das "Ink-Bleeding"-Verhalten der Proben visuell beurteilt.

In Tabelle 2 sind erfindungsgemäße Beispiele mit "EB" und Vergleichsbeispiele mit "VB" bezeichnet. Die ermittelten Meßwerte und Beurteilungen ergeben sich aus Tabelle 2:

**Tabelle 2:**

| Beispiel | Pigment | Tintenfixiermittel | Viskosität der Streichmasse [Brookfield (Spindel 2 / 50 rpm / 25°C)] | Flächenbezogene Masse der Tintenempfangsschicht [g/m²] | "Ink-Bleeding"-Verhalten |
|---|---|---|---|---|---|
| 10 VB | 1 | A | > 800 | 8,4 | Schlecht |
| 11 EB | 1 | B | 536 | 8,2 | Noch Gut |
| 12 VB | 1 | C | > 800 | 8,6 | Schlecht |
| 13 VB | 2 | A | 408 | 7,8 | Schlecht |
| 14 EB | 2 | B | 344 | 8,8 | Gut |
| 15 VB | 2 | C | > 800 | 8,7 | Schlecht |
| 16 VB | 3 | A | 352 | 7,8 | Unbefriedigend |
| 17 EB | 3 | B | 264 | 8,3 | Gut |
| 18 VB | 3 | C | > 800 | 8,8 | Schlecht |
| 19 VB | 4 | A | 672 | 8,0 | Schlecht |
| 20 EB | 4 | B | 440 | 8,1 | Gut |
| 21 VB | 4 | C | > 800 | 8,1 | Schlecht |
| 22 VB | 5 | A | 208 | 8,3 | Sehr Schlecht |
| 23 VB | 5 | B | 144 | 8,0 | Schlecht |
| 24 VB | 5 | C | 720 | 8,1 | Schlecht |

Ausgehend von der Grundrezeptur 2 und den Tintenempfangsschicht-Komponenten gemäß Beispiel 17 wurde das Tintenfixiermittel-Pigment-Verhältnis variiert. Die so hergestellten Proben sollen hinsichtlich ihres "Ink-Bleeding"-Verhaltens und der Naßwischfestigkeit aufgebrachter Druckbilder untersucht werden.
Zur Untersuchung dieser Naßwischfestigkeit werden die Druckbilder, an denen zuvor das "Ink-Bleeding"-Verhalten der Proben beurteilt wurde, mit Wasser beträufelt. Nach 2 Sekunden Einwirkzeit wischt man mehrmals mit einem Finger unter gleichbleibendem Druck über das Druckbild. Dabei sollte das Druckbild möglichst wenig, im Idealfall überhaupt nicht verwischt werden.

### Beispiele 17, 26, 27 und Vergleichsbeispiele 25, 28:

Für das Vergleichsbeispiel 25 und das erfindungsgemäße Beispiel 26 wird zur Reduzierung des Tintenfixiermittelanteils ― bezogen auf 500 Gew.-Teile (lutro) der Grundrezeptur .2 ― eine geringere Menge an Tintenfixiermittel und somit eine größere Menge aller anderen Komponenten unter ständigem Rühren in einem Behälter eingetragen. Für das erfindungsgemäße Beispiel 27 und das Vergleichsbeispiel 28 wird zur Erhöhung des Tintenfixiermittelanteils ― bezogen auf 500 Gew.-Teile (lutro) der Grundrezeptur 2 ― eine größere Menge an Tintenfixiermittel und somit eine geringere Menge aller anderen Komponenten unter ständigem Rühren in einem Behälter eingetragen.
Die sich anschließende Herstellung der Proben und das jeweilige Aufbringen eines Kontrolldruckes geschieht entsprechend obiger Ausführungen.

Tabelle 3 gibt die eingestellten Tintenfixiermittel-Pigment-Verhältnisse und das jeweils visuell beurteilte "Ink-Bleeding"-Verhalten der Proben sowie die Naßwischfestigkeit der aufgebrachten Druckbilder wieder. Erneut sind erfindungsgemäße Beispiele mit "EB" und Vergleichsbeispiele mit "VB" bezeichnet:

**Tabelle 3:**

| Beispiel | | Tintenfixiermittel-Pigment-Verhältnisse | "Ink-Bleeding"-Verhalten | Naßwischfestigkeit |
|---|---|---|---|---|
| 25 | VB | 1 : 10 | Schlecht | Sehr gut |
| 26 | EB | 1 : 5 | Gut | Gut |
| 17 | EB | 1 : 4,53 | Gut | Gut |
| (siehe auch Tabelle 2) | | | | |
| 27 | EB | 1 : 3,3 | Gut | Befriedigend |
| 28 | VB | 1 : 1,5 | Gut | Schlecht |

Die erfindungsgemäßen Beispiele veranschaulichen besonders deutlich, daß es mit dem erfindungsgemäßen Aufzeichnungsmaterial überzeugend gelungen ist, ein kostengünstiges Aufzeichnungsmaterial für das Tintenstrahl-Druckverfahren zur Verfügung zu stellen, das insbesondere bei Bedruckung mit Aufzeichnungstinten auf Pigmentbasis eine hervorragende Fixierung der aufgebrachten Tinten garantiert und so das sogenannte "Ink-Bleeding" reduziert, was bei den Vergleichsbeispielen nicht gegeben ist. Die erfindungsgemäßen Beispiele zeigen ferner, daß das neue Aufzeichnungsmaterial eine akzeptable Naßwischfestigkeit aufgebrachter Druckbilder sicherstellt, wozu die Vergleichsbeispiele nicht in der Lage sind.

## Patentansprüche

1. Aufzeichnungsmaterial für das Tintenstrahl-Druckverfahren mit einem Substrat und einer Tintenempfangsschicht, die auf mindestens eine Seite des Substrates aufgebracht ist, wobei die Tintenempfangsschicht ein anorganisches Pigment und ein Tintenfixiermittel enthält, das mindestens zwei Substanzen umfaßt, von denen die erste Substanz ein Polyethylaminepichlorhydrin und die zweite Substanz ein mehrwertiges Metallsalz ist, **dadurch gekennzeichnet, dass**
• die erste Substanz ein mittelmolekular verzweigtes Polyamin-Epichlorhydrin-Kondensationsprodukt ist,
• das Verhältnis der ersten Substanz zur zweiten Substanz zwischen 3 : 1 und 16 : 1 liegt,
• das Pigment zu mindestens 80 Gew.-% mit einer D₅₀-Teilchengröße (Malvern) in einem Bereich von 4 µm einschließlich bis 12 µm einschließlich vorliegt und
• das Verhältnis Tintenfixiermittel zu Pigment in einem Bereich von einschließlich 1 : 2 bis einschließlich 1 : 6 liegt. bezogen auf die Gewichtsprozente von Pigment und Tintenfixiermittel in der Tintenempfangsschicht.

2. Aufzeichnungsmaterial nach Anspruch 1, **dadurch gekennzeichnet, dass** die zweite Substanz ein aus der Magnesium-Chlorid, Aluminium-Chlorid und CalciumChlorid umfassende Gruppe ausgewähltes mehrwertiges Metallsalz ist.

3. Aufzeichnungsmaterial nach einem der Ansprüche 1 oder 2, **dadurch gekennzeichnet, dass** das Verhältnis der ersten Substanz zur zweiten Substanz zwischen 6 : 1 und 14 : 1 beträgt.

4. Aufzeichnungsmaterial nach einem der Ansprüche 1 bis 3, **dadurch gekennzeichnet, dass** das Pigment zu mindestens 80 Gew.-% mit einer D₅₀-Teilchengröße (Malvern) in einem Bereich zwischen 6 und 12 µm vorliegt.

5. Aufzeichnungsmaterial nach einem der Ansprüche 1 bis 4, **dadurch gekennzeichnet, dass** das Pigment ausgewählt ist aus der Gruppe, umfassend Aluminiumhydroxid, gefällte Kieselsäure und Kieselgel.

6. Aufzeichnungsmaterial nach einem der Ansprüche 1 bis 5, **dadurch gekennzeichnet, dass** das Verhältnis Tintenfixiermittel zu Pigment in einem Bereich von einschließlich 1 : 3 bis einschließlich 1 : 5,5 liegt.

7. Aufzeichnungsmaterial nach einem der Ansprüche 1 bis 6, **dadurch gekennzeichnet, dass** die Tintenempfangsschicht mindestens ein Bindemittel enthält ausgewählt aus der Gruppe, umfassend Polyvinylalkohol, Styrolbutadienlatex, Polyacrylate und Polymerdispersionen aus Ethylen-Vinylacetat.

8. Aufzeichnungsmaterial nach einem der Ansprüche 1 bis 7, **dadurch gekennzeichnet, dass** zwischen Substrat und Tintenempfangsschicht mindestens eine Präparationsschicht angeordnet ist.

9. Aufzeichnungsmaterial nach einem der Ansprüche 1 bis 8, **dadurch gekennzeichnet, dass** die Tintenempfangsschicht aus zwei übereinander positionierten Lagen besteht.

10. Verfahren zur Aufzeichnung nach dem diskontinuierlichen Tintenstrahldruck-Verfahren mit einer Aufzeichnungstinte auf Pigmentbasis und mit einem Aufzeichnungsmaterial nach einem der Ansprüche 1 bis 9.

## Claims

1. Recording material for the inkjet printing method, comprising a substrate and an ink-receiving layer that is applied to at least one side of the substrate, the ink-receiving layer containing an inorganic pigment and an ink-fixing agent that consists of at least two substances, of which the first substance is a polyethylamine epichlorohydrin and the second substance is a polyvalent metal salt, **characterised in that**
• the first substance is a middle molecular weight branched polyamine-epichlorohydrin condensation product,
• the ratio of the first substance to the second substance is between 3 : 1 and 16 : 1,
• the pigment accounts for at least 80% by weight with a D₅₀ particle size (Malvern) ranging from 4 µm inclusive to 12 µm inclusive and
• the ratio of ink-fixing agent to pigment is in a range from 1 : 2 inclusive to 1 : 6 inclusive, based on the percentages by weight of pigment and ink-fixing agent in the ink-receiving layer.

2. Recording material according to claim 1, **characterised in that** the second substance is a polyvalent metal salt selected from the group consisting of magnesium chloride, aluminium chloride and calcium chloride.

3. Recording material according to one of claims 1 and 2, **characterised in that** the ratio of the first substance to the second substance is between 6 : 1 and 14: 1.

4. Recording material according to any one of claims 1 to 3, **characterised in that** the pigment accounts for at least 80 % by weight with a D₅₀ particle size (Malvern) in a range between 6 and 12 µm.

5. Recording material according to any one of claims 1 to 4, **characterised in that** the pigment is selected from the group consisting of aluminium hydroxide, precipitated silicic acid and silica gel.

6. Recording material according to any one of claims 1 to 5, **characterised in that** the ratio of ink-fixing agent to pigment is in a range from 1 : 3 inclusive to 1 : 5.5 inclusive.

7. Recording material according to any one of claims 1 to 6, **characterised in that** the ink-receiving layer contains at least one binding agent selected from the group consisting of polyvinyl alcohol, styrene-butadiene latex, polyacrylates and polymer dispersions of ethylene vinyl acetate.

8. Recording material according to any one of claims 1 to 7, **characterised in that** at least one preparation layer is arranged between substrate and ink-receiving layer.

9. Recording material according to any one of claims 1 to 8, **characterised in that** the ink-receiving layer consists of two superimposed layers.

10. Method for recording in accordance with the discontinuous inkjet printing method using a pigment-based recording ink and using a recording material according to any one of claims 1 to 9.

## Revendications

1. Matériau d'enregistrement pour le procédé d'impression à jet d'encre, comprenant un substrat et une couche de réception de l'encre qui est déposée sur au moins une face du substrat, la couche de réception de l'encre contenant un pigment inorganique et un agent de fixation de l'encre, lequel contient au moins deux substances dont la première substance est une polyéthylamine-épichlorhydrine et la deuxième substance est un sel métallique polyvalent, **caractérisé en ce que**
- la première substance est un produit de condensation de polyamine-épichlorhydrine ramifié à poids moléculaire moyen,
- le rapport de la première substance à la deuxième substance est compris entre 3 : 1 et 16 : 1,
- le pigment est présent pour au moins 80 % en poids avec une taille de particules D₅₀ (Malvern) dans l'intervalle de 4 µm inclus jusqu'à 12 µm inclus et
- le rapport de l'agent de fixation de l'encre au pigment est situé dans un intervalle compris entre 1 : 2 inclus et 1 : 6 inclus, rapporté aux pourcentages en poids du pigment et de l'agent de fixation de l'encre dans la couche de réception de l'encre.

2. Matériau d'enregistrement selon la revendication 1, **caractérisé en ce que** la deuxième substance est un sel métallique polyvalent choisi dans le groupe comprenant le chlorure de magnésium, le chlorure d'aluminium et le chlorure de calcium.

3. Matériau d'enregistrement selon une des revendications 1 ou 2, **caractérisé en ce que** le rapport de la première substance à la deuxième substance est compris entre 6 : 1 et 14 : 1.

4. Matériau d'enregistrement selon une des revendications 1 à 3, **caractérisé en ce que** le pigment est présent pour au moins 80 % en poids avec une taille de particules D₅₀ (Malvern) situé dans un intervalle compris entre 6 et 12 µm.

5. Matériau d'enregistrement selon une des revendications 1 à 4, **caractérisé en ce que** le pigment est choisi dans le groupe consistant en l'hydroxyde l'aluminium, l'acide silicique précipité et le gel de silice.

6. Matériau d'enregistrement selon une des revendications 1 à 5, **caractérisé en ce que** le rapport de l'agent de fixation de l'encre au pigment est situé dans un intervalle compris entre 1 : 3 inclus et 1 : 5,5 inclus.

7. Matériau d'enregistrement selon une des revendications 1 à 6, **caractérisé en ce que** la couche de réception de l'encre contient au moins un liant choisi dans le groupe consistant en l'alcool polyvinylique, latex de styrène-butadiène, les polyacrylates et les dispersions de polymères à base d'éthylène-acétate de vinyle.

8. Matériau d'enregistrement selon une des revendications 1 à 7, **caractérisé en ce qu'**au moins une couche de préparation est interposée entre le substrat et la couche de réception de l'encre.

9. Matériau d'enregistrement selon une des revendications 1 à 8, **caractérisé en ce que** la couche de réception de l'encre est composée de deux strates positionnées l'une au-dessus de l'autre.

10. Procédé pour enregistrer selon le procédé d'impression à jet d'encre discontinu, à l'aide d'une encre d'enregistrement à base de pigment et un matériau d'enregistrement selon l'une des revendications 1 à 9.
